# EUROPEAN PATENT SPECIFICATION

(11) **EP 1 457 212 B1**
(45) Date of publication and mention of the grant of the patent: **07.03.2007**
(21) Application number: 02805476.5
(22) Date of filing: 18.12.2002
(51) Int. Cl.: A61K 47/46, A61K 47/06, A61K 47/10, A61K 47/18, A61P 27/04

(54) **EYE DROPS**
AUGENTROPFEN
GOUTTES OPHTALMIQUES

(30) Priority: 21.12.2001 JP 2001390152
(43) Date of publication of application: 15.09.2004
(73) Proprietor: Senju Pharmaceutical Co., Ltd., Osaka-shi, Osaka 541-0046 (JP)
(72) Inventor: NAKAYAMA, Hisayuki, Nishinomiya-shi, Hyogo 622-0826 (JP); NEMOTO, Fukiko, Kobe-shi, Hyogo 651-2109 (JP)
(74) Representative: von Kreisler, Alek
(86) International application number: PCT/JP2002/013263
(87) International publication number: WO 2003/053472

(56) References cited:
- EP-A- 1 016 406
- JP-A- 10 306 022
- JP-A- 11 180 858
- JP-A- 2001 122 774
- JP-A- 2001 187 733
- JP-A- 2001 322 936
- PATENT ABSTRACTS OF JAPAN vol. 1999, no. 12, 29 October 1999 (1999-10-29) & JP 11 180858 A (ROHTO PHARMACEUT CO LTD), 6 July 1999 (1999-07-06)
- PATENT ABSTRACTS OF JAPAN vol. 1999, no. 10, 31 August 1999 (1999-08-31) & JP 11 130667 A (SENJU PHARMACEUT CO LTD), 18 May 1999 (1999-05-18)
- PATENT ABSTRACTS OF JAPAN vol. 1997, no. 09, 30 September 1997 (1997-09-30) & JP 09 132526 A (LION CORP), 20 May 1997 (1997-05-20)

## Description

### Technical Field

The present invention relates to eye drops containing a clove perfume and a peppermint perfume.

### Background Art

Though a wide variety of eye drops are marketed which contain refreshing agents like menthol, camphor, borneol, etc. for refreshment of the eye, there still is a demand for eye drops which give an improved sensation when they are instilled. On the other hand, among various ingredients generally contained in eye drops, some have peculiar chemical smells that would sometimes give an uncomfortable sensation upon instillation of the eye drops, such as glutathione (a compound possessing an SH group), sorbic acid and salts thereof, dipotassium glycyrrhizinate, camphor, borneol, surfactants, acetic acid and salt thereof.

The objective of the present invention is to provide eye drops that give an improved sensation upon instillation and to provide a method to suppress peculiar smells of ingredients contained in eye drops. The term "a sensation upon instillation" means the potency of refreshing sensation, the duration of refreshing sensation and the quality (briskness, breeziness, pervasiveness and depth) of refreshing sensation and/or fragrance, etc.

Document JP 11180858 discloses eye drops which contain a refrigerant selected from a group consisting of 1-menthol, DL-menthol, D-camphor, DL-camphor, D-borneol, DL-borneol, geraniol, eucalyptus oil, bergamot oil, fennel oil, peppermint oil, rose oil and cool mint, and one or more surfactants. The surfactants belong to the group of nonionic and amphiphatic surface active agents.

### Disclosure of Invention

As a result of studies focused on attaining the above objective, the inventors of the present invention surprisingly found that simultaneous addition of a clove perfume, which has been used in preparations for dental external or oral use, in combination with a peppermint perfume, made it possible to provide such eye drops that give improved sensation upon instillation, and to suppress peculiar chemical smells of ingredients in eye drops. The present invention was completed through further studies based thereon.

Thus, the present invention provides, (1) eye drops comprising a clove perfume and a peppermint perfume, and (2) eye drops comprising, in addition to a clove perfume and a peppermint perfume, a eucalyptus perfume and/or lemon perfume. The present invention also provides (3) eye drops of (1) or (2) above and further comprising a terpenoid, and (4) those eye drops further comprising a soothing agent. The present invention further provides (5) eye drops containing the above ingredients and suitable for eyes wearing a contact lens. The present invention also provides a method for suppressing peculiar chemical smells of eye drops comprising addition of a clove perfume and a peppermint perfume.

### Best Mode for Carrying Out the Invention

In the present invention, a clove perfume may be obtained, for example, by steam distillation of dried flower bud of clove (Eugenia caryophyllata), a Myrtaceae plant ("Koryo no Jissai Chishiki", 2nd Ed., Forth Issue, p. 34, April 26, 1999, TOYO KEIZAI INC.). It may also be obtained by any one of other extraction methods such as hot-water distillation, organic solvent extraction, mechanical expression, involatile solvent extraction, or supercritical fluid extraction. Any of these extraction methods may also be used to obtain not only a clove perfume but also a peppermint perfume, a eucalyptus perfume, and a lemon perfume mentioned below.

A clove perfume contains a variety of characteristic ingredients like eugenol, its acetic ester, vanillin, caryophyllene, etc. Such characteristic ingredients contained in a clove perfume may be collected by means of steam distillation, hot-water distillation, organic solvent extraction, mechanical expression, involatile solvent extraction, supercritical fluid extraction, etc. and added to eye drops. Alternatively, eugenol, which is the primary ingredient among the ingredients in a clove perfume, may be added as a clove perfume to eye drops. In this case, either eugenol extracted by means of steam distillation, hot-water distillation, organic solvent extraction, mechanical expression, involatile solvent extraction, supercritical fluid extraction, as mentioned above, or synthetic eugenol may be used.

A peppermint perfume may be obtained, for example, by steam distillation of the whole plant of foreign species of peppermint (Mentha piperita var. vulgaris) of mint family ("Koryo no Jissai Chishiki", 2nd Ed., Forth Issue, p. 54, April 26, 1999, TOYO KEIZAI INC.). It may also be obtained by any one of other extraction methods such as hot-water distillation, organic solvent extraction, mechanical expression, involatile solvent extraction, or supercritical fluid extraction.

A peppermint perfume contains a variety of characteristic ingredients such as menthol, menthyl esters, menthone, menthofuran, jasmone, etc. Such characteristic ingredients contained in a peppermint perfume may be collected by means of steam distillation, hot-water distillation, organic solvent extraction, mechanical expression, involatile solvent extraction, supercritical fluid extraction, etc. and added to eye drops.

Alternatively, menthol, which is the primary ingredient among the ingredients in a peppermint perfume, may be added as a peppermint perfume to eye drops. In this case, either menthol extracted by means of steam distillation, hot-water distillation, organic solvent extraction, mechanical expression, involatile solvent extraction, supercritical fluid extraction, as mentioned above, or synthetic menthol may be used.

The combined content of a clove perfume and a peppermint perfume in the eye drops of the present invention is 0.0001-2 (W/V)%, preferably 0.001-1 (W/V)%, and more preferably 0.005-0.3 (W/V)% [0.005-0.1 (W/V)% where no soothing agent such as chlorobutanol is contained, and 0.01-0.3 (W/V)% where a soothing agent such as chlorobutanol is contained] of the total content of the eye drops. And the weight ratio of the content of a peppermint perfume to that of a clove perfume is 1:0.01-100, preferably 1:0.05-30, and more preferably 1:0.1-15.

The eye drops of the present invention may further contain at lease one perfume selected from the group consisting of a eucalyptus perfume and a lemon perfume. For example, a combination of a clove perfume, a peppermint perfume and a eucalyptus perfume, and a combination of a clove perfume, a peppermint perfume and a lemon perfume may be employed. Eye drops that give further improved sensation upon instillation may be obtained based on a combination of the four, a clove perfume, a peppermint perfume, a eucalyptus perfume and a lemon perfume.

A eucalyptus perfume and a lemon perfume may be obtained by means of steam distillation, hot-water distillation, organic solvent extraction, physical expression, involatile solvent extraction, supercritical fluid extraction, etc. of, e.g., a fresh leaves of Eucalyptus globus Labillardiere, a Myrtaceae plant, or the fruit skin of a lemon (Citrus limon Burum f.), a Rutaceae plant., respectively. It is also possible to use a perfume made of synthetic compounds which are characteristic of these perfumes.

When three perfumes, a clove perfumes, a peppermint perfumes and a eucalyptus perfume, are employed in combination, their total content in the whole eye drops is 0.00011-2.5 (W/V)%, preferably 0.0015-1.3 (W/V)%, and more preferably 0.006-0.4 (W/V)% [0.006-0.1 (W/V)% where no soothing agent like chlorobutanol is contained, and 0.01-0.4 (W/V)% where a soothing agent like chlorobutanol is contained]. And the weight ratio of the content of a peppermint perfume to that of a clove perfume is 1:0.01-100, preferably 0.05-30, and more preferably 1:0.1-15. The weight ratio of a eucalyptus perfume to a clove perfume is 1:0.01-20, preferably 1:0.5-10, and more preferably 1:0.1-5.

Where three perfumes, a clove perfume, a peppermint perfume and a lemon perfume, are employed in combination, their total content in the whole eye drops is 0.00011-2.5 (W/V)%, preferably 0.0015-1.3 (W/V)%, and more preferably 0.006-0.4 (W/V)% [0.006-0.1 (W/V)% where no soothing agent like chlorobutanol is contained, and 0.01-0.4 (W/V)% where a soothing agent like chlorobutanol is contained]. And the weight ratio of the content of a peppermint perfume to that of a clove perfume is 1:0.01-100, preferably 1:0.05-30, and more preferably 1:0.1-15. And the weight ratio of the content of a lemon perfume to that of a clove perfume is 1:0.01-20, preferably 1:0.5-10, and more preferably 1:0.1-5.

Where four perfumes, a clove perfume, a peppermint perfume, a eucalyptus perfume and a lemon perfume, are employed in combination, their total content in the whole eye drops is 0.00012-3 (W/V)%, preferably 0.002-1.6 (W/V)%, and more preferably 0.007-0.5 (W/V)% [0.007-0.1 (W/V)% where no soothing agent like chlorobutanol is contained, and 0.01-0.5 (W/V)% where a soothing agent like chlorobutanol is contained]. And the weight ratio of the content of a peppermint perfume to that of a clove perfume is 1:0.01-100, preferably 1:0.05-30, and more preferably 1:0.1-15. The weight ratio of the content of a eucalyptus perfume to that of a clove perfume is 1:0.01-20, preferably 1:0.5-10, and more preferably 1:0.1-5, and the weight ratio of the content of a lemon perfume to that of a clove perfume is 1:0.01-20, preferably 1:0.5-10, and more preferably 1:0.1-5.

In addition to the above-mentioned four perfumes, the eye drops of the present invention may further contain aliphatic higher alcohols, esters, phenol ethers, ketones, lactones, aromatic aldehydes, etc.

The aforementioned perfumes, both natural or synthetic, to be employed in the present invention are commercially available from perfume manufacturers and can be employed without any processing.

The eye drops of the present invention may further contain a refreshing agent such as a terpenoid. Examples of terpenoids include monoterpenes such as menthol, camphor, borneol, geraniol, menthone, cineole, limonene, citral, pinene, linalol, fenchyl alcohol, ionone, safranal, terpinene and the like. Among these, menthol, camphor, borneol, geraniol, menthone, cineol and limonene are particularly preferred. Any one of these may be employed alone, or two or more of them in combination as desired. The content of terpenoids in the whole eye drops is preferably 0.0005-0.1 (W/V)%, and more preferably 0.001-0.06 (W/V)%.

Further, the eye drops of the present invention may also contain a soothing agent. As a soothing' agent, a surface anesthetic agent, a topical anesthetic agent or an agent causing topical numbness may be used, such as chlorobutanol, dibucaine hydrochloride, tetracaine hydrochloride, ethyl aminobenzoate, lidocaine, lidocaine hydrochloride and procaine hydrochloride. Any one of them may be employed alone, or two or more of them in combination. The content of a soothing agent is preferably 0.01-1 (W/V)%, and particularly preferably 0.05-0.5 (W/V)%.

In the present invention, a clove perfume and a peppermint perfume contained significantly reduce (mask) peculiar chemical smells of those ingredients that are often contained in eye drops, such as glutathione (a compound possessing an SH group), sorbic acid and salts thereof, dipotassium glycyrrhizinate, camphor, borneol, surfactants, acetic acid and salt thereof.

Further, the eye drops of the present invention are also suitable for instillation to the eyes wearing a variety of contact lenses such as a non-oxygen gas permeable hard contact lens, an oxygen gas permeable hard contact lens and a soft contact lens. Among these, instillation is made with particular advantage to the eyes wearing a soft contact lens.

The eye drops of the present invention may, as needed, also contain other ingredients that are generally contained in eye drops. Examples of such ingredients include solubilizing agent, buffers, isotonizers, preservatives, stabilizers, suspending agents, surfactants, chelating agents, thickening agents, pH adjusting agents, etc., and they may be added at a conventional amount insofar as they do not interfere with the effect of the present invention.

Examples of solubilizing agents include polysorbate 80, polyvinylpyrrolidone, polyethylene glycol, propylene glycol, polyoxyethylenehydrogenated castor oil 60, polyoxyl stearate 40.

Where one or more buffers are added to the eye drops of the present invention, the resulting pH of the eye drops is preferably 4-9, and more preferably 5-8. Examples of buffers added include, for example, boric acid or its salts such as borax, citric acid or its salts such as a sodium salt, tartaric acid or its salt such as a sodium salt, gluconic acid or its salt such as a sodium salt, acetic acid or its salts such as a sodium salt, phosphoric acid, disodium hydrogen phosphate, sodium dihydrogen phosphate or the like, and a variety of amino acids.

Any isotonizers may be chosen without limitation insofar as they are water soluble and exert no adverse influence like irritation to the eye. Examples include sorbitol, glucose, mannitol, glycerol, propylene glycol, sodium chloride, potassium chloride.

Examples of preservatives include p-hydroxy benzoate esters, benzalkonium chloride, benzethonium chloride, chlorobutanol, benzyl alcohol, sorbic acid or its salts, chlorhexidine gluconate, sodium dehydroacetate, cetylpyridinium chloride, alkyldiaminoethylglycine hydrochloride.

Examples of stabilizers include ascorbic acid, sodium edetate, cyclodextrin, condensed phosphoric acid or its salts, sulfite salts, citric acid or its salts.

Examples of suspending agents include methylcellulose, hydroxypropylcellulose, polyvinylpyrrolidone, polyoxyethylenehydrogenated castor oil 60, polyoxyl stearate 40, polyethylene glycol, sodium carboxymethylcellulose, polyvinylalcohol.

As surfactants, any type of surfactants, i.e., nonionic surfactants, anionic surfactants, amphoteric surfactants and cationic surfactants, may be employed.

Examples of anionic surfactants include sodium lauroyl sarcosinate, lauroyl-L-glutamic acid triethanolamine and sodium myristyl sarcosinate and the like. Examples of amphoteric surfactants include lauryldimethylaminoacetic acid betaine, 2-alkyl-N-carboxymethyl-N-hydroxy-ethylimidazolinium betaine, alkyldiaminoglycine hydrochloride and the like. Examples of nonionic surfactants include polysorbate 80, polyoxyethylenehydrogenated castor oil 60, polyoxyl stearate 40, polyoxyethylene lauryl ether and the like. Examples of cationic surfactants include benzethonium chloride, benzalkonium chloride, cetylpyridinium chloride and the like.

Examples of chelating agents include sodium edetate, sodium citrate, condensed phosphoric acid or its salts (condensed phosphates) and the like.

Examples of thickening agents include methylcellulose, hydroxyethylcellulose, hydroxypropylcellulose, hydroxypropylmethylcellulose, sodium chondroitin sulfate, sodium carboxymethylcellulose, polyvinylpyrrolidone, polyvinylalcohol, polyethylene glycol and the like.

Examples of pH adjusting agents include sodium hydroxide, potassium hydroxide, sodium carbonate, hydrochloric acid, phosphoric acid, citric acid, acetic acid and the like.

### [Examples]

The present invention will be described below in further detail with reference to test examples and working examples.

### [Test Example] Test of Sensation upon Application (1)

Test method: According to the in-company Manual for Application Test of OTC Drugs based on Good Clinical Practice (GCP), Preparation 1 of the present invention and Preparation 1 for Comparison both shown in Table 1, which had been manufactured according to the GMP for Investigational New Drugs were instilled (2 drops/eye), to one of the both eyes of nine adults who had been confirmed to be without any local abnormality by a responsible doctor from the nominated medical institution, and evaluation was conducted according to the criteria shown below. The test was carried out twice. In the second test, instillation was made to the opposite eye to the one that received instillation in the first test. After the test, the responsible doctor of the nominated medical institution confirmed that no eye of the subjects showed any local abnormality.

### Evaluation Criteria:

### Potency of Refreshing Sensation

5: Potent
4: Somewhat potent
3: Moderate
2: Somewhat weak
1: Weak
Fragrance
5: Good
4: Somewhat good
3: Moderate
2: Somewhat bad
1: Bad
0: No

### Sensation upon Instillation

The number of the panelists who answered that they would like to chose the indicated eye drops if they were to receive instillation again.

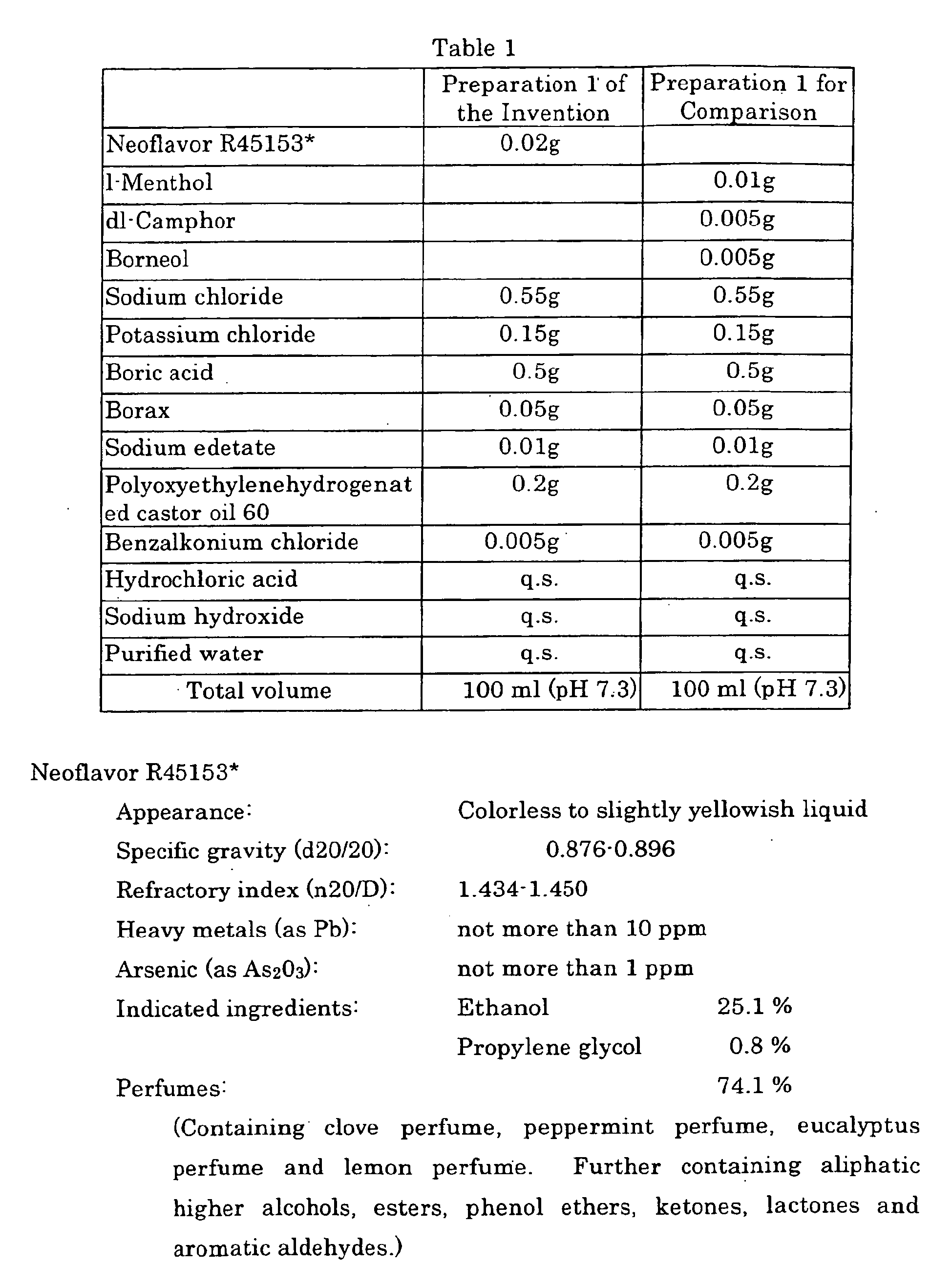

Test results : The results of the test of Preparation 1 of the present invention and Preparation 1 for Comparison are shown in Table 2. It is seen from the test results that the Preparation 1 of the present invention containing a clove perfume and a peppermint perfume is comparative in potency of the refreshing sensation to Preparation 1 for Comparison containing terpenoids, which are conventionally employed ingredients as refreshing agents in eye drops, but is better than the Preparation 1 for Comparison in fragrance and sensation upon instillation.

**Table 2**

| | Preparation 1 of the Invention | Preparation 1 for Comparison |
|---|---|---|
| Potency of refreshing sensation (mean) | 3.5 | 3.4 |
| Fragrance (mean) | 3.7 | 2.6 |
| Sensation upon instillation | 11 | 7 |

### [Test Example] Test of Sensation upon Application (2)

Test Method: According to the in-company Manual for Application Test of OTC Drugs based on Good Clinical Practice (GCP), preparation 2 of the present invention and Preparation 2 for Comparison both shown in Table 3, which had been manufactured according to the GMP for Investigational New Drugs were instilled (2 drops/eye), to one of the both eyes of nine adults who had been confirmed to be without any local abnormality by a responsible doctor from the nominated medical institution, and evaluation was conducted according to the criteria shown below. The test was carried out twice. In the second test, instillation was made to the opposite eye to the one that received instillation in the first test. After the test, the responsible doctor of the nominated medical institution confirmed that no eye of the subjects showed any local abnormality.

### Evaluation Criteria:

### Potency of Refreshing Sensation

5: Potent
4: Somewhat potent
3: Moderate
2: Somewhat weak
1: Weak

### Duration of Refreshing Sensation

The length of time from just after instillation to when refreshing sensation ceased (measured with a stopwatch)

### Quality of the Refreshing Sensation (briskness, breeziness, pervasiveness and depth)

The number of the panelists who chose the indicated eye drops as being better with regard to each of the indicated items.

### Fragrance

5: Good
4: Somewhat good
3: Moderate
2: Somewhat bad
1: Bad
0: No

### Sensation upon Instillation

The number of the panelists who answered that they would like to chose the indicated eye drops if they were to receive instillation again.

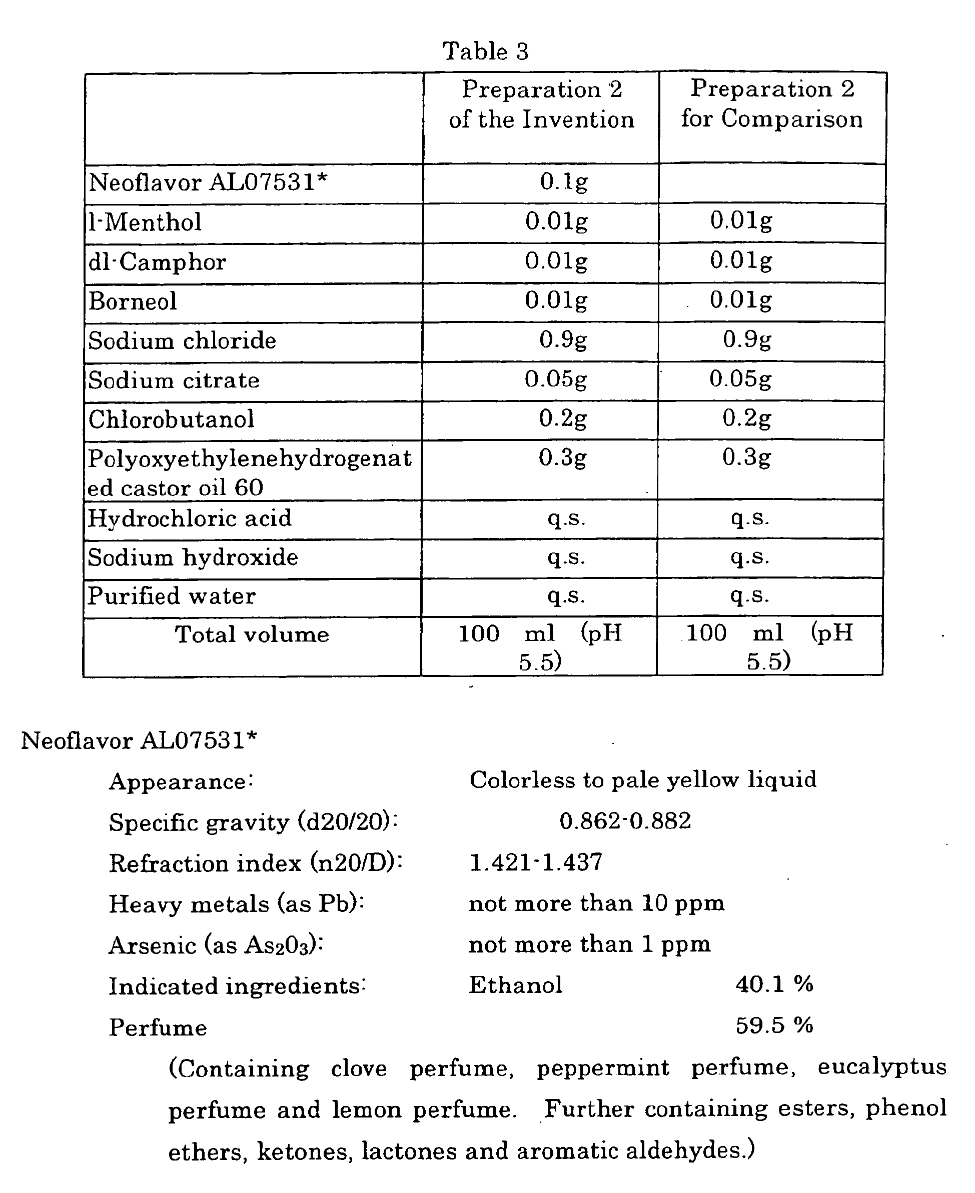

Test results : The results of the test of Preparation 2 of the present invention and Preparation 2 for Comparison are shown in Table 4. It is seen from the test results that the Preparation 2 of the present invention containing a clove perfume, a pepper perfume together with terpenoids is superior to Preparation 2 for Comparison containing terpenoids alone, in all the items, i.e., potency of the refreshing sensation upon instillation, duration of the refreshing sensation, quality of the refreshing sensation (briskness, breeziness, pervasiveness and depth), fragrance and sensation upon instillation.

**Table 4**

| | | Preparation 2 of the Invention | Preparation 2 for Comparison |
|---|---|---|---|
| Potency of refreshing sensation (mean) | | 3.1 | 2.1 |
| Duration of refreshing sensation (mean) | | 281.7 sec | 202.8 sec |
| Quality of refreshing sensation | Briskness | 12 | 4 |
| | Breeziness | 13 | 2 |
| | Pervasiveness | 14 | 1 |
| | Depth | 8 | 2 |
| Fragrance (mean) | | 3.9 | 2.6 |
| Sensation upon instillation | | 14 | 4 |

Eye drops of the present invention were prepared below by a conventional method.

### [Example 1]

| | |
|---|---|
| Naphazoline hydrochloride | 0.003 g |
| Neostigmine methylsulfate | 0.005 g |
| Allantoin | 0.1 g |
| Chlorpheniramine maleate | 0.03 g |
| Pyridoxine hydrochloride | 0.1 g |
| Potassium L-aspartate | 1 g |
| Sodium chondroitin sulfate | 0.1 g |
| Boric acid | 1.1 g |
| Sodium citrate | 0.1 g |
| Chlorobutanol | 0.2 g |
| Benzalkonium chloride solution (10 W/V%) | 0.05 mL |
| Neoflavor AL07531* | 0.07 g |
| 1-Menthol | 0.047 g |
| Polyoxyethylenehydrogenated castor oil 60 | 0.2 g |
| Hydrochloric acid | q.s. |
| Sodium hydroxide | q.s. |
| Sterile purified water | q.s. |
| | Total volume 100 ml, pH 5.5 |

| | |
|---|---|
| Neoflavor AL075311*: Refer to Table 3. | |

In about 70 ml of purified water were sequentially dissolved chlorobutanol, boric acid, sodium citrate, naphazoline hydrochloride, neostigmine methylsulfate, allantoin, chlorpheniramine maleate, pyridoxine hydrochloride, potassium L-aspartate, sodium chondroitin sulfate, benzalkonium chloride solution (10 W/V%). To the solution were added Neoflavor ALo7531 and 1-menthol which had been dispersed by heating with polyoxyethylenehydrogenated castor oil 60, and the pH of the solution was adjusted to 5.5 with hydrochloric acid or sodium hydroxide. Purified water then was added to make the total volume of 100 ml. After sterilization by filtration, the solution was filled in 15-ml polyethylene terephthalate containers.

### [Example 2]

| | |
|---|---|
| Sodium chloride | 0.45 g |
| Potassium chloride | 0.15 g |
| Boric acid | 0.5 g |
| Borax | 0.045 g |
| Hydroxypropylmethylcellulose | 0.2 g |
| Clove perfume | 0.0034 g |
| Peppermint perfume | 0.025 g |
| Polysorbate 80 | 0.2 g |
| Benzalkonium chloride solution (10 W/V%) | 0.05 ml |
| Purified water | q.s. |
| | Total volume 100 ml, pH 7.3 |

Hydroxypropylmethylcellulose was dispersed in about 70 ml of warmed purified water and allowed to cool to dissolve. In this solution were sequentially dissolved potassium chloride, boric acid, borax and benzalkonium chloride solution (10 W/V%), and the clove perfume and the peppermint perfume that had been dispersed by heating with polysorbate 80 were then added. Purified water then were added to make the total volume of 100 ml. After sterilization by filtration, the solution was filled in 15-ml polyethylene terephthalate containers.

### [Example 3]

| | |
|---|---|
| Glucose | 0.005 g |
| Sodium chloride | 0.5 g |
| Potassium chloride | 0.15 g |
| Calcium chloride | 0.015 g |
| Sodium citrate | 0.2 g |
| Boric acid | 0.45 g |
| Borax | 0.02 g |
| Clove perfume | 0.005 g |
| Peppermint perfume | 0.005 g |
| Eucalyptus perfume | 0.005 g |
| Polyoxyl stearate 40 | 0.2 g |
| Benzalkonium chloride solution (10 W/V%) | 0.05 ml |
| Purified water | q.s. |
| | Total volume 100 ml, pH 7.3 |

In about 70 ml of purified water were sequentially dissolved sodium chloride, potassium chloride, calcium chloride, glucose, sodium citrate, boric acid, borax and benzalkonium chloride solution (10 W/V%). To the solution then were added the clove perfume, the peppermint perfume and the eucalyptus perfume which had been dispersed by heating with polyoxyl stearate 40. Purified water then were added to make the total volume of 100 ml. After sterilization by filtration, the solution was filled in 15-ml polyethylene terephthalate containers.

### [Example 4]

| | |
|---|---|
| Tetrahydrozoline hydrochloride | 0.05 g |
| Chlorpheniramine maleate | 0.03 g |
| Dipotassium glycyrrhizinate | 0.1 g |
| d-α-tocopherol acetate | 0.05 g |
| Sodium L-glutamate | 0.2 g |
| Sodium chloride | 0.9 g |
| Chlorobutanol | 0.2 g |
| Clove perfume | 0.01 g |
| Peppermint perfume | 0.13 g |
| Lemon perfume | 0.01 g |
| 1-Menthol | 0.005 g |
| Polyoxyethylenehydrogenated castor oil 60 | 0.3 g |
| Benzalkonium chloride solution (10 WN%) | 0.05 ml |
| Hydrochloric acid | q.s. |
| Sodium hydroxide | q.s. |
| Purified water | q.s. |
| | Total volume 100 ml, pH 5.5 |

In about 70 ml of purified water were sequentially dissolved chlorobutanol, tetrahydrozoline hydrochloride, chlorpheniramine maleate, dipotassium glycyrrhizinate, sodium L-glutamate, sodium chloride and benzalkonium chloride solution (10 W/V%). To the solution were added d-α-tocopherol acetate which had been dispersed by heating with half amount of the polyoxyethylenehydrogenated castor oil 60, and then the clove perfume, the peppermint perfume, the lemon perfume and 1-menthol dispersed by heat with the remaining half of the polyoxyethylenehydrogenated castor oil 60. The pH of the solution was adjusted to 5.5 with hydrochloric acid or sodium hydroxide. Purified water then was added to make the total volume of 100 ml. After sterilization by filtration, the solution was filled in 15-ml polyethylene terephthalate containers.

### [Example 5]

| | |
|---|---|
| Naphazoline hydrochloride | 0.003 g |
| Neostigmine methylsulfate | 0.005 g |
| Chlorpheniramine maleate | 0.03 g |
| Cyanocobalamin | 0.01 g |
| Pyridoxine hydrochloride | 0.05 g |
| Potassium L-aspartate | 1.0 g |
| Sodium citrate | 0.2 g |
| Sodium chloride | 0.7 g |
| Chlorobutanol | 0.2 g |
| Clove perfume | 0.05 g |
| Peppermint perfume | 0.2 g |
| Eucalyptus perfume | 0.025 g |
| Lemon perfume | 0.025 g |
| dl-Camphor | 0.003 g |
| Polyoxyethylenehydrogenated castor oil 60 | 0.3 g |
| Benzalkonium chloride solution (10 W/V%) | 0.05 ml |
| Hydrochloric acid | q.s. |
| Sodium hydroxide | q.s. |
| Purified water | q.s. |
| | Total volume 100 ml, pH 5.5 |

In about 70 ml of purified water were sequentially dissolved chlorobutanol, naphazoline hydrochloride, neostigmine methylsulfate, chlorpheniramine maleate, cyanocobalamin, pyridoxine hydrochloride, potassium L-aspartate, sodium citrate, sodium chloride and benzalkonium chloride solution (10 W/V%). To the solution were added the clove perfume, the peppermint perfume, the eucalyptus perfume, the lemon perfume and dl-camphor which had been dispersed by heating with polyoxyethylenehydrogenated castor oil 60. The pH of the solution was adjusted to 5.5 with hydrochloric acid or sodium hydroxide. Purified water then was added to make the total volume of 100 ml. After sterilization by filtration, the solution was filled in 15-ml polyethylene terephthalate containers.

### [Example 6]

| | |
|---|---|
| Naphazoline hydrochloride | 0.003 g |
| Neostigmine methylsulfate | 0.005 g |
| Allantoin | 0.1 g |
| Chlorpheniramine maleate | 0.03 g |
| Pyridoxine hydrochloride | 0.1 g |
| Potassium L-aspartate | 1 g |
| Boric acid | 1.1 g |
| Sodium citrate | 0.1 g |
| Chlorobutanol | 0.2 g |
| Benzalkonium chloride solution (10 W/V%) | 0.05 ml |
| Neoflavor AL07531* | 0.07 g |
| 1-Menthol | 0.047 g |
| Polyoxyethylenehydrogenated castor oil 60 | 0.2 g |
| Hydrochloric acid | q.s. |
| Sodium hydroxide | q.s. |
| Purified water | q.s. |
| | Total volume 100 ml, pH 5.5 |

| | |
|---|---|
| Neoflavor AL07531*: Refer to Table 3. | |

In about 70 ml of purified water were sequentially dissolved chlorobutanol, boric acid, sodium citrate, naphazoline hydrochloride, neostigmine methylsulfate, allantoin, chlorpheniramine maleate, pyridoxine hydrochloride, potassium L-aspartate and benzalkonium chloride solution (10 W/V%). To the solution were added Neoflavor AL07531 and 1-mentol which had been dispersed by heating with polyoxyethylenehydrogenated castor oil 60. The pH of the solution was adjusted to 5.5 with hydrochloric acid or sodium hydroxide. Purified water then was added to make the total volume of 100 ml. After sterilization by filtration, the solution was filled in 9-ml polyethylene terephthalate containers.

### Industrial Applicability

The eye drops of the present invention, because of a clove perfume and a peppermint perfume contained, give improved sensation upon instillation. They also have improved flagrance. Thus, in combination with their effect of suppressing peculiar chemical smells of eye-drops' ingredients, such eye drops are provided that have good fragrance and improved sensation upon instillation.

## Claims

1. Eye drops comprising a clove perfume and a peppermint perfume.

2. The eye drops of claim 1 further comprising at least one perfume selected from the group consisting of a eucalyptus perfume and a lemon perfume.

3. The eye drops of claim 1 or 2 further comprising one or more terpenoids.

4. The eye drops of claim 3 wherein the terpenoid is at least one compound selected from the group consisting of menthol, borneol and camphor.

5. The eye drops of one of claims 1 to 4 further comprising a soothing agent.

6. The eye drops of claim 5 wherein the soothing agent is at least one compound selected from the group consisting of chlorobutanol, dibucaine hydrochloride, tetracaine hydrochloride, ethyl aminobenzoate, lidocaine, lidocaine hydrochloride and procaine hydrochloride.

7. Eye drops comprising a clove perfume, a peppermint perfume, a eucalyptus perfume and a lemon perfume.

8. The eye drops of claim 7 further comprising one or more terpenoids.

9. The eye drops of claim 8 wherein the terpenoid is at least one compound selected from the group consisting of menthol, borneol and camphor.

10. The eye drops of one of claims 7 to 9 further comprising a soothing agent

11. The eye drops of claim 10 wherein the soothing agent is at least one compound selected from the group consisting of chlorobutanol, dibucaine hydrochloride, tetracaine hydrochloride, ethyl aminobenzoate, lidocaine, lidocaine hydrochloride and procaine hydrochloride.

12. The eye drops of one of claims 1 to 11 wherein the eye drops is for the eye wearing a contact lens.

13. The eye drops of claim 12 wherein the contact lens is a soft contact lens.

14. A method for suppressing chemical smells of eye drops comprising adding a clove perfume and a peppermint perfume to the eye drop.

15. The method of claim 14 further comprising adding at least one perfume selected from the group consisting of a eucalyptus perfume and a lemon perfume

## Patentansprüche

1. Augentropfen, die einen Gewürznelken-Duftstoff und einen Pfefferminz-Duftstoff umfassen.

2. Augentropfen gemäß Anspruch 1, die weiterhin wenigstens einen Duftstoff umfassen, der aus der Gruppe ausgewählt ist, die aus einem Eukalyptus-Duftstoff und einem Zitronen-Duftstoff besteht.

3. Augentropfen gemäß Anspruch 1 oder 2, die weiterhin ein oder mehrere Terpenoide umfassen.

4. Augentropfen gemäß Anspruch 3, wobei es sich bei dem Terpenoid um wenigstens eine Verbindung handelt, die aus der Gruppe ausgewählt ist, die aus Menthol, Borneol und Campher besteht.

5. Augentropfen gemäß einem der Ansprüche 1 bis 4, die weiterhin ein Linderungsmittel umfassen.

6. Augentropfen gemäß Anspruch 5, wobei es sich bei dem Linderungsmittel um wenigstens eine Verbindung handelt, die aus der Gruppe ausgewählt ist, die aus Chlorbutanol, Dibucain-Hydrochlorid, Tetracain-Hydrochlorid, Ethylaminobenzoat, Lidocain, Lidocain-Hydrochlorid und Procain-Hydrochlorid besteht.

7. Augentropfen, die einen Gewürznelken-Duftstoff, einen Pfefferminz-Duftstoff, einen Eukalyptus-Duftstoff und einen Zitronen-Duftstoff umfassen.

8. Augentropfen gemäß Anspruch 7, die weiterhin ein oder mehrere Terpenoide umfassen.

9. Augentropfen gemäß Anspruch 8, wobei es sich bei dem Terpenoid um wenigstens eine Verbindung handelt, die aus der Gruppe ausgewählt ist, die aus Menthol, Borneol und Campher besteht.

10. Augentropfen gemäß einem der Ansprüche 7 bis 9, die weiterhin ein Linderungsmittel umfassen.

11. Augentropfen gemäß Anspruch 10, wobei es sich bei dem Linderungsmittel um wenigstens eine Verbindung handelt, die aus der Gruppe ausgewählt ist, die aus Chlorbutanol, Dibucain-Hydrochlorid, Tetracain-Hydrochlorid, Ethylaminobenzoat, Lidocain, Lidocain-Hydrochlorid und Procain-Hydrochlorid besteht.

12. Augentropfen gemäß einem der Ansprüche 1 bis 11, wobei die Augentropfen für Kontaktlinsen tragende Augen bestimmt sind.

13. Augentropfen gemäß Anspruch 12, wobei die Kontaktlinse eine weiche Kontaktlinse ist.

14. Verfahren zur Unterdrückung von chemischen Gerüchen von Augentropfen, das das Hinzufügen eines Gewürznelken-Duftstoffs und eines Pfefferminz-Duftstoffs zu den Augentropfen umfasst.

15. Verfahren gemäß Anspruch 14, das weiterhin das Hinzufügen wenigstens eines Duftstoffs umfasst, der aus der Gruppe ausgewählt ist, die aus einem Eukalyptus-Duftstoff und einem Zitronen-Duftstoff besteht.

## Revendications

1. Gouttes ophtalmiques comprenant un parfum de clou de girofle et un parfum de menthe poivrée.

2. Gouttes ophtalmiques selon la revendication 1, comprenant en outre au moins un parfum sélectionné dans le groupe constitué d'un parfum d'eucalyptus et d'un parfum de citron.

3. Gouttes ophtalmiques selon la revendication 1 ou 2, comprenant en outre un ou plusieurs terpènoïdes.

4. Gouttes ophtalmiques selon la revendication 3, où le terpènoïde est au moins un composé sélectionné dans le groupe constitué du menthol, du bornéol et du camphre.

5. Gouttes ophtalmiques selon l'une des revendications 1 à 4, comprenant en outre un agent apaisant.

6. Gouttes ophtalmiques selon la revendication 5, où l'agent apaisant est au moins un composé sélectionné dans le groupe constitué du chlorobutanol, du chlorhydrate de dibucaïne, du chlorhydrate de tétracaïne, de l'aminobenzoate d'éthyle, de la lidocaïne, du chlorhydrate de lidocaïne et du chlorhydrate de procaïne.

7. Gouttes ophtalmiques comprenant un parfum de clou de girofle, un parfum de menthe poivrée, un parfum d'eucalyptus et un parfum de citron.

8. Gouttes ophtalmiques selon la revendication 7, comprenant en outre un ou plusieurs terpènoïdes.

9. Gouttes ophtalmiques selon la revendication 8, où le terpènoïde est au moins un composé sélectionné dans le groupe constitué du menthol, du bornéol et du camphre.

10. Gouttes ophtalmiques selon l'une des revendications 7 à 9, comprenant en outre un agent apaisant.

11. Gouttes ophtalmiques selon la revendication 10, où l'agent apaisant est au moins un composé sélectionné dans le groupe constitué du chlorobutanol, du chlorhydrate de dibucaïne, du chlorhydrate de tétracaïne, de l'aminobenzoate d'éthyle, de la lidocaïne, du chlorhydrate de lidocaïne et du chlorhydrate de procaïne.

12. Gouttes ophtalmiques selon l'une des revendications 1 à 11, où les gouttes opthalmiques sont destinées au port oculaire de lentilles de contact.

13. Gouttes ophtalmiques selon la revendication 12, où la lentille de contact est une lentille de contact souple.

14. Procédé pour supprimer les odeurs de produits chimiques dégagées par les gouttes opthalmiques, comprenant l'addition d'un parfum de clou de girofle et d'un parfum de menthe poivrée aux gouttes ophtalmiques.

15. Procédé selon la revendication 14, comprenant en outre l'addition d'au moins un parfum sélectionné dans le groupe constitué d'un parfum d'eucalyptus et d'un parfum de citron.
